# EUROPÄISCHE PATENTSCHRIFT

(11) **EP 1 523 895 B1**
(45) Veröffentlichungstag und Bekanntmachung des Hinweises auf die Patenterteilung: **07.03.2007**
(21) Anmeldenummer: 03023469.4
(22) Anmeldetag: 18.10.2003
(51) Int. Cl.: A23L 1/30

(54) **Zubereitungen zur oralen Aufnahme**
Composition for oral administration
Composition pour administration orale

(43) Veröffentlichungstag der Anmeldung: 20.04.2005
(73) Patentinhaber: Cognis IP Management GmbH, 40589 Düsseldorf (DE)
(72) Erfinder: Rull Prous, Santiago, Barcelona 08034 (ES); Blasquez, José Fernandez, Terrassa 08228 (ES); Escudero, Joaquin, Barcelona 08015 (ES); Fabry, Dr. Bernd, Korschenbroich 41352 (DE)

(56) Entgegenhaltungen:
- EP-A- 1 175 901
- US-B1- 6 423 325
- US-B1- 6 630 163
- DATABASE FSTA [Online] INTERNATIONAL FOOD INFORMATION SERVICE (IFIS), FRANFURT/MAIN, DE; GIL M I ET AL: "Changes in pomegranate juice pigmentation during ripening." Database accession no. 95-1-08-h0121 XP002274168 & JOURNAL OF THE SCIENCE OF FOOD AND AGRICULTURE 1995 CORRESPONDENCE (REPRINT) ADDRESS, F. A. TOMAS-BARBERAN, DEP. DE CIENCIA Y TEC. DE ALIMENTOS, CEBAS, CSIC, PO BOX 4195, MURCIA 30080, SPAIN, Bd. 68, Nr. 1, Seiten 77-81,
- DATABASE FSTA [Online] INTERNATIONAL FOOD INFORMATION SERVICE (IFIS), FRANFURT/MAIN, DE; VIVAS DE GAULEJAC N ET AL: "Identification of procyanidin A2 in grape and wine of Vitis vinifera L. cv. Merlot Noir and Cabernet Sauvignon." Database accession no. 2001-00-h1631 XP002272879 & JOURNAL INTERNATIONAL DES SCIENCES DE LA VIGNE ET DU VIN 35 (1) 51-56 2001 CORRESPONDENCE (REPRINT) ADDRESS, N. VIVAS, CENT. D'ETUDE STRUCTURALE ET D'ANALYSE DES MOLECULES ORGANIQUES, UNIV. BORDEAUX I, 33405 TALENCE, FRANCE. E-MAIL N.VIVAS(A)CESAMO.U,

## Beschreibung

### Gebiet der Erfindung

Die Erfindung befindet sich auf dem Gebiet der Nahrungsmittelzusatz- bzw. - ergänzungsstoffe und betrifft neue Zubereitungen zur oralen Aufnahme, enthaltend spezielle Pflanzenextrakte und ausgewählte mehrfach ungesättigte Fettsäuren.

### Stand der Technik

In den letzten Jahren hat der Markt für Nahrungsmittelzusatzstoffe einen ungeheuren Aufschwung erfahren. Vom Verbraucher werden sowohl Produkte gewünscht, die in einem eher undifferenzierten Ansatz den körperlichen Wohlbefinden nutzen und die Abwehrkräfte steigern, wie dies beispielsweise typisch für Vitamine ist, als auch solche, die unter den Begriffen "Health Food" oder "Dietary Supplements" bekannt sind und beispielsweise den Fettabbau oder den Muskelaufbau beschleunigen. So wird beispielsweise in der internationalen Patentanmeldung **WO 97/46230** (WARF) vorgeschlagen, konjugierte Linolsäure für diesen Zweck einzusetzen. Ein weiteres Beispiel für den wachsenden Markt für Nahrungsmittelergänzungsstoffe kann unter der Überschrift "Cosmetic inside" zusammengefasst werden. Hier geht es darum, insbesondere Alterungserscheinungen der Haut durch orale Aufnahme von geeigneten Wirkstoffen entgegenzuwirken. Lange bekannt für solche Anwendungen für Carotinoide.

Des weiteren sei auf die Druckschrift **US 6,630,163** (Murad) verwiesen, aus der ein Mittel zur Verbesserung der Hautbeschaffenheit bekannt ist, welches u.a. einen Granatapfelextrakt und darüber hinaus optional noch OPC-haltige Traubenkernextrakte sowie omega-3-Fettsäuren aufweisen kann. Aus der **EP 1175901 A1** (Kaneka) sind Mittel zur Behandlung von Fettleibigkeit bekannt, welche Triglyceride auf Basis von konjugierten Fettsäuren enthalten. Schließlich wird in der **US 6,423,325** (Alaluf) eine Zubereitung zur Hautpflege vorgeschlagen, welche neben einer Mischung von Petroselinsäure und konjugierter Linolsäure auch Granatapfelextrakt enthalten kann.

In diesem Zusammenhang besteht jedoch das Bedürfnis, Zubereitungen zur Verfügung zu stellen, die einerseits die einzelnen Aufgabenstellungen im Vergleich zum Stand der Technik in verbesserter Weise lösen - also beispielsweise die gleichen Effekte bei geringerer Dosierung ergeben - als auch unterschiedliche Aufgabenstellungen miteinander verbinden.

Die Aufgabe der vorliegenden Erfindung hat darin bestanden neue Zubereitungen zur oralen Aufnahme zur Verfügung zu stellen, die im Sinne von Nahrungsmittelergänzungsstoffen die kosmetische Beschaffenheit von Haut, Haaren und Fingernägeln verbessern und die Gewichtsregulierung unterstützen.

### Beschreibung der Erfindung

Gegenstand der Erfindung sind Zubereitungen zur oralen Aufnahme, enthaltend
(a) Extrakte der Pflanze *Punica granatum* bzw. deren Wirkstoffe und
(b) konjugierte Linolsäure (CLA), deren Ester oder Glyceride, bei denen der Acylrest wenigstens 30 Gew.-% t10,c12-Isomere, wenigstens 30 Gew.-% c9,t11-Isomere und in Summe weniger als 1 Gew.-% 8,10-, 11,13- und t,t-Isomere aufweist.

Überraschenderweise wurde gefunden, dass die delphinidin- bzw. delphinidin-3,5-diglucosidreichen Punica-Extrakte in Kombination mit konjugierter Linolsäure einer speziellen Isomerenverteilung bzw. deren Derivaten bei oraler Verabreichung zu einer synergistisch verbesserten Lipogenase-Inhibierung führen und die Flüssigkeitsdrainage steigern. Diese Effekte kann man sich zur Nutze machen, um einerseits die den Abbau von Körperfetten, z.B. im Rahmen einer Diät zu unterstützen, als auch den Flüssigkeitshaushalt der Haut zu regulieren und dabei im wesentlichen die Symptome einer trockenen Haut zu bekämpfen. Gleichzeitig wird das Haarwachstum stimuliert und den Ursachen des Haarausfalls und brüchiger Fingernägel entgegengewirkt.

### Extrakte der Punica granatum

Unter der Bezeichnung *Punica granatum* verbirgt sich der Granatapfel, ein ursprünglich in Asien heimischer, leuchtend rot blühender Strauch oder Baum, der schon im Altertum kultiviert worden ist. Die Extrakte des Granatapfels enthalten 12 bis 13 Gew.-% Zucker (hauptsächlich Invertzucker), 0,5 bis 1 Gew.-% Fruchtsäuren, speziell Zitronensäure sowie als Essenz des roten Farbstoffes das physiologisch aktive Delphinidin-1,2-glykosid bzw. dessen Aglykon.

### Konjugierte Linolsäure

Als Komponente (b) gelangt konjugierte Linolsäure (CLA), deren Ester - speziell solche mit niederen aliphatischen Alkoholen mit 1 bis 4 Kohlenstoffatomen - oder deren Glyceride, speziell die synthetischen Triglyceride zum Einsatz. Dabei handelt es sich um bekannte Stoffe, die üblicherweise durch basenkatalysierte Isomerisierung von Distelöl oder entsprechenden Alkylestern und nachfolgende enzymatische Hydrolyse hergestellt werden. Die CLA-Spezies haben dabei eine Spezifikation zu erfüllen, gemäß der der Acylrest wenigstens 30 Gew.-% t10,c12-Isomere, wenigstens 30 Gew.-% c9,t11-Isomere und in Summe weniger als 1 Gew.-% 8,10-, 11,13- und t,t-Isomere aufweist. Entsprechende Produkte sind beispielsweise unter der Bezeichnung Tonalin® CLA-80 (Cognis) im Handel.

### Extraktion

Die Herstellung sowohl der delphinidin(glykosid)-haltigen als auch der proanthocyanilidinhaltigen Extrakte kann in an sich bekannter Weise erfolgen, d.h. beispielsweise durch wässrigen, alkoholischen oder wässrig-alkoholischen Auszug der Pflanzen bzw. Pflanzenteile bzw. der Blätter oder Früchte. Geeignet sind alle herkömmlichen Extraktionsverfahren wie z.B. Mazeration, Remazeration, Digestion, Bewegungsmazeration, Wirbelextraktion, Ultraschallextraktion, Gegenstromextraktion, Perkolation, Reperkolation, Evakolation (Extraktion unter vermindertem Druck), Diakolation oder Festflüssig-Extraktion unter kontinuierlichem Rückfluss. Für den großtechnischen Einsatz vorteilhaft ist die Perkolationsmethode. Als Ausgangsmaterial können frische Pflanzen oder Pflanzenteile eingesetzt werden, üblicherweise wird jedoch von getrockneten Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Gefriermahlung genannt. Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, verwendet werden. Besonders bevorzugt ist die Extraktion mit Methanol, Ethanol, Pentan, Hexan, Heptan, Aceton, Propylenglykolen, Polyethylenglykolen sowie Ethylacetat sowie Mischungen hieraus sowie deren wässrige Gemische. Die Extraktion erfolgt in der Regel bei 20 bis 100 °C, bevorzugt bei 30 bis 90 °C, insbesondere bei 60 bis 80 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem beliebigen Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffinenge) bei der Extraktion getrockneter Blätter liegen im Bereich von 3 bis 15, insbesondere 6 bis 10 Gew.-%. Die vorliegende Erfindung umfasst die Erkenntnis, dass die Extraktionsbedingungen sowie die Ausbeuten der Endextrakte vom Fachmann ja nach gewünschtem Einsatzgebiet gewählt werden können. Diese Extrakte, die in der Regel Aktivsubstanzgehalte (= Feststoffgehalte) im Bereich von 0,5 bis 10 Gew.-% aufweisen, können als solche eingesetzt werden, es ist jedoch ebenfalls möglich, das Lösungsmittel durch Trocknung, insbesondere durch Sprüh- oder Gefriertrocknung vollständig zu entfernen. Die Extrakte können auch als Ausgangsstoffe für die Gewinnung der oben genannten reinen Wirkstoffe dienen, sofern diese nicht auf synthetischem Wege einfacher und kostengünstiger hergestellt werden können. Demzufolge kann der Wirkstoffgehalt in den Extrakten 5 bis 100, vorzugsweise 50 bis 95 Gew.-% betragen. Die Extrakte selbst können als wässrige und/oder in organischen Solventien gelöste Zubereitungen sowie als sprüh- bzw. gefriergetrocknete, wasserfreie Feststoffe vorliegen. Als organische Lösungsmittel kommen in diesem Zusammenhang beispielsweise die aliphatischen Alkohole mit 1 bis 6 Kohlenstoffatomen (z.B. Ethanol), Ketone (z.B. Aceton), Halogenkohlenwasserstoffe (z.B. Chloroform oder Methylenchlorid), niedere Ester oder Polyole (z.B. Glycerin oder Glycole) in Frage.

Vorzugsweise werden die Komponenten (a) und (b) jeweils im Gewichtsverhältnis 90:10 bis 10:90 eingesetzt, wobei besondere synergistische Effekte im Bereich von 75:25 bis 25:75 und insbesondere 60:40 bis 40:60 zu beobachten sind.

### Verkapselung

In einer besonderen Ausführungsform der vorliegenden Erfindung werden die oralen Zubereitungen in verkapselter Form - beispielsweise in Gestalt üblicher Gelatinemakrokapseln - vorzugsweise aber in mikroverkapselter Form eingesetzt.

Unter den Begriffen "Mikrokapsel" oder "Nanokapsel" werden vom Fachmann sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

Mikrokapseln und Verfahren zu ihrer Herstellung sind Gegenstand früherer Patenanmeldungen der Patentanmelderin **[WO 01/01926, WO 01/01927, WO 01/01928, WO 01/01929].**

Mikrokapseln mit mittleren Durchmessern im Bereich von 0,0001 bis 5, vorzugsweise 0,001 bis 0,5 und insbesondere 0,005 bis 0,1 mm, bestehend aus einer Hüllmembran und einer die Wirkstoffe enthaltenden Matrix, können beispielsweise erhalten werden, indem man
(a1) aus Gelbildnern, kationischen Polymeren und Wirkstoffen eine Matrix zubereitet,
(a2) gegebenenfalls die Matrix in einer Ölphase dispergiert,
(a3) die dispergierte Matrix mit wässrigen Lösungen anionischer Polymere behandelt und gegebenenfalls dabei die Ölphase entfernt.
oder
(b1) aus Gelbildnem, anionischen Polymeren und Wirkstoffen eine Matrix zubereitet,
(b2) gegebenenfalls die Matrix in einer Ölphase dispergiert,
(b3) die dispergierte Matrix mit wässrigen Kationpolymerlösungen behandelt und gegebenenfalls dabei die Ölphase entfernt;
oder
(c1) aus Gelbildnern und Wirkstoffen eine Matrix zubereitet,
(c2) die Matrix mit einer Kationpolymerlösung versetzt und
(c3) die Mischung auf einen pH-Wert einstellt der oberhalb des pKₛ-Wertes des Kationpolymers liegt;
oder
(d1) wässrige Wirkstoffzubereitungen mit Ölkörpern in Gegenwart von Emulgatoren zu O/W-Emulsionen verarbeitet,
(d2) die so erhaltenen Emulsionen mit wässrigen Lösungen anionischer Polymere behandelt,
(d3) die so erhaltene Matrix mit wässrigen Kationpolymerlösungen in Kontakt bringt und
(d4) die so erhaltenen Verkapselungsprodukte von der wässrigen Phase abtrennt;
oder
den Wirkstoff abwechselnd mit Schichten aus unterschiedlich geladenen Polyelektrolyten einhüllt (layer-by-layer-Technologie).
- Gelbildner
   Im Sinne der Erfindung werden als Gelbildner vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3- und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.
- Kationische Polymere
   Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol. Vorzugsweise wird als Verkapselungsmaterial Chitosan eingesetzt. Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten: 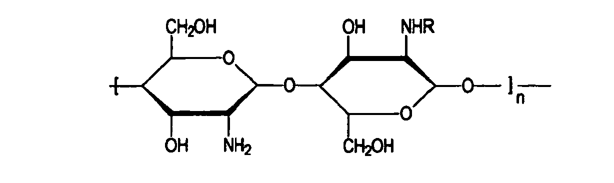
   Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.
- Ölphase
   Die Matrix kann vor der Bildung der Membran optional in einer Ölphase dispergiert werden. Als Öle kommen für diesen Zweck beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglycerid-mischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.
- Anionpolymere
   Die anionische Polymere haben die Aufgabe, mit den Chitosanen Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein: 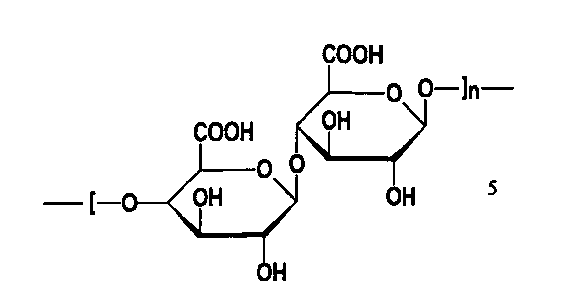
   Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.
- **Emulgatoren**
   Als Emulgatoren kommen anionische, amphotere, kationische oder vorzugsweise nichtionische oberflächenaktive Verbindungen aus mindestens einer der folgenden Gruppen in Frage:
   - Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
   - Alkyl- und/oder Alkenyloligoglykoside mit 8 bis 22 Kohlenstoffatomen im Alk(en)ylrest und deren ethoxylierte Analoga;
   - Anlagerungsprodukte von 1 bis 15 Mol Ethylenoxid an Ricinusöl und/oder gehärtetes Ricinusöl;
   - Anlagerungsprodukte von 15 bis 60 Mol Ethylenoxid an Ricinusöl und/oder gehärtetes Ricinusöl;
   - Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
   - Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
   - Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
   - Mono-, Di- und Trialkylphosphate sowie Mono-, Di- und/oder Tri-PEGalkylphosphate und deren Salze;
   - Wollwachsalkohole;
   - Polysiloxan-Polyalkyl-Polyether-Copolymere bzw. entsprechende Derivate;
   - Block-Copolymere z.B. Polyethylenglycol-30 Dipolyhydroxystearate;
   - Polymeremulgatoren, z.B. Pemulen-Typen (TR-1,TR-2) von Goodrich;
   - Polyalkylenglycole,
   - Glycerincarbonat,
   - aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure, sowie
   - Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat.
- Herstellverfahren Mikrokapseln
   Zur Herstellung der Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100°C, wird eine zweite wässrige Lösung zugegeben, welche das Kationpolymer, vorzugsweise das Chitosan in Mengen von 0,1 bis 2, vorzugsweise 0,25 bis 0,5 Gew.-% und den Wirkstoffen in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzuzugeben. Nach der Herstellung der Matrix aus Gelbildner, Kationpolymer und Wirkstoffen kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des Kationpolymers in der Matrix mit den anionischen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Anionpolymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 0,01 bis 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die Anionpolymere auch zur Herstellung der Matrix einsetzen und die Verkapselung mit den Kationpolymeren, speziell den Chitosanen durchführen. Alternativ kann die Verkapselung auch unter ausschließlicher Verwendung von Kationpolymeren erfolgen, wobei man sich deren Eigenschaft zu Nutze macht, bei pH-Werten oberhalb des pKs-Wertes zu koagulieren.
   In einem zweiten alternativen Verfahren wird zur Herstellung der erfindungsgemäßen Mikrokapseln wird zunächst eine O/W-Emulsion zubereitet, welche neben dem Ölkörper, Wasser und den Wirkstoffen eine wirksame Menge Emulgator enthält. Zur Herstellung der Matrix wird diese Zubereitung unter starkem Rühren mit einer entsprechenden Menge einer wässrigen Anionpolymerlösung versetzt. Die Membranbildung erfolgt durch Zugabe der Chitosanlösung. Der gesamte Vorgang findet vorzugsweise im schwach sauren Bereich bei pH = 3 bis 4 statt. Falls erforderlich erfolgt die pH-Einstellung durch Zugabe von Mineralsäure. Nach der Membranbildung wird der pH-Wert auf 5 bis 6 angehoben, beispielsweise durch Zugabe von Triethanolamin oder einer anderen Base. Hierbei kommt es zu einem Anstieg der Viskosität, die durch Zugabe von weiteren Verdickungsmitteln, wie z.B. Polysacchariden, insbesondere Xanthan-Gum, Guar-Guar, Agar-Agar, Alginaten und Tylosen, Carboxymethylcellulose und Hydroxyethylcellulose, höhermolekularen Polyethylenglycolmono- und -diestern von Fettsäuren, Polyacrylaten, Polyacrylamiden und dergleichen noch unterstützt werden kann. Abschließend werden die Mikrokapseln von der wässrigen Phase beispielsweise durch Dekantieren, Filtrieren oder Zentrifugieren abgetrennt.
   In einem dritten alternativen Verfahren erfolgt die Bildung der Mikrokapseln um einen vorzugsweise festen, beispielsweise kristallinen Kern, indem dieser schichtweise mit entgegengesetzt geladenen Polyelektrolyten eingehüllt wird. In diesem Zusammenhang sei auf das Europäische Patent **EP 1064088 B1** (Max-Planck Gesellschaft) verwiesen.

### Gewerbliche Anwendbarkeit

Die erfindungsgemäßen Zubereitungen zeigen bei oraler Aufnahme eine synergistisch verbesserte Inhibierung der Lipogenasetätigkeit und der Drainagefunktion in der Haut. Ein weiterer Gegenstand der Erfindung betrifft daher die Verwendung von Mischungen, enthaltend
(a) Extrakte der Pflanze *Punica granatum* bzw. deren Wirkstoffe und
(b) konjugierte Linolsäure (CLA), deren Ester oder Glyceride enthalten, bei denen der Acylrest wenigstens 30 Gew.-% t10,c12-Isomere, wenigstens 30 Gew.-% c9,t11-Isomere und in Summe weniger als 1 Gew.% 8,10-, 11,13- und t,t-Isomere aufweist.
zur Herstellung von Nahrungsmittelzusatzstoffen, speziell zur Verminderung des Körperfettes im menschlichen oder tierischen Organismus sowie zur Regulierung des Feuchtigkeitsgehaltes in der Haut.

### Beispiele

### Beispiel 1

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer Lösung von 10 g Glycerin 90 ml Wasser und dann mit einer Zubereitung von 2,5 g Natriumalginat in Form einer 10 Gew.-%igen wässrigen Lösung, 1 g konjugierte Linolsäure (Tonalin® CLA 80), 1 g getrockneter *Punica granatum* Extrakt, 0,5 g Phenonip® und 0,5 g Polysorbat-20 (Tween® 20, ICI) in 64 g Wasser versetzt. Die erhaltene Matrix wurde filtriert, auf 60 °C erwärmt und in eine 1 Gew.-%ige Lösung von Chitosanglycolat in Wasser getropft. Zum Erhalt von Mikrokapseln gleichen Durchmessers wurden die Zubereitungen anschließend gesiebt.

### Beispiel 2

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer Lösung von 10 g Glycerin 90 ml Wasser und dann mit einer Zubereitung von 2,5 g Natriumalginat in Form einer 10 Gew.-%igen wässrigen Lösung, 1 g konjugierte Linolsäure (Tonalin® CLA-80), 1 g getrockneter *Punica granatum* Extrakt, 1 g getrockneter *Lichi chinensis* Extrakt, 0,5 g Phenonip® und 0,5 g Polysorbat-20 (Tween® 20, ICI) in 64 g Wasser versetzt. Die erhaltene Matrix wurde filtriert, auf 60 °C erwärmt und in eine 1 Gew.-%ige Lösung von Chitosanglycolat in Wasser getropft. Zum Erhalt von Mikrokapseln gleichen Durchmessers wurden die Zubereitungen anschließend gesiebt.

## Patentansprüche

1. Zubereitungen zur oralen Aufnahme, enthaltend
(a) Extrakte der Pflanze *Punica granatum* bzw. deren Wirkstoffe und
(b) konjugierte Linolsäure (CLA), deren Ester oder Glyceride, bei denen der Acylrest wenigstens 30 Gew.-% t10,c12-Isomere, wenigstens 30 Gew.-% c9,t11-Isomere und in Summe weniger als 1 Gew.-% 8,10-, 11,13- und t,t-Isomere aufweist.

2. Zubereitungen nach Anspruch 1, **dadurch gekennzeichnet, dass** sie als Wirkstoffe der Komponente (a) Delphinidin und/oder Delphinidin-3,5-diglykosid enthalten.

3. Zubereitungen nach den Ansprüchen 1 und/oder 2, **dadurch gekennzeichnet, dass** sie die Komponenten (a) und (b) im Gewichtsverhältnis 90:10 bis 10: 0 enthalten.

4. Zubereitungen nach mindestens einem der Ansprüche 1 bis 3, **dadurch gekennzeichnet, dass** sie in verkapselter Form vorliegen.

5. Verwendung von Mischungen gemäß Anspruch 1 zur Herstellung von Nahrungsmittelzusatzstoffen.

## Claims

1. Preparations for oral administration containing
(a) extracts of the plant *Punica granatum* or the active constituents thereof and
(b) conjugated linoleic acid (CLA), its esters or glycerides, where the acyl group contains at least 30% by weight t10,c12 isomers, at least 30% by weight c9, t11 isomers and, in all, less than 1% by weight 8,10-, 11,13- and t,t isomers.

2. Preparations as claimed in claim 1, **characterized in that** they contain delphinidin and/or delphinidin-3,5-diglycoside as the active constituents of component (a).

3. Preparations as claimed in claims 2 and/or 3, **characterized in that** they contain components (a) and (b) in a ratio by weight of 90:10 to 10:0.

4. Preparations as claimed in at least one of claims 1 to 3, **characterized in that** they are present in encapsulated form.

5. Use of the mixtures claimed in claim 1 for the production of food additives.

## Revendications

1. Préparations pour administration orale contenant :
(a) des extraits de la plante *Punica granatum* ou ses principes actifs, et
(b) de l'acide linoléique conjugué (CLA), ses esters ou glycérides, dont le radical acyle présente au moins 30 % en poids d'isomères -t10,c12, au moins 30 % en poids d'isomères -c9,t11 et, au total, moins de 1 % en poids d'isomères -8,10, -11,13 et -t,t.

2. Préparations selon la revendication 1,
**caractérisées en ce que**
comme principes actifs du composant (a), elles contiennent de la delphinidine et/ou du 3,5-diglycoside de delphinidine.

3. Préparations selon les revendications 1 et/ou 2,
**caractérisées en ce qu'**
elles contiennent les composants (a) et (b) dans le rapport pondéral 90:10 à 10:0.

4. Préparations selon au moins l'une des revendications 1 à 3,
**caractérisées en ce qu'**
elles se présentent sous forme encapsulée.

5. Utilisation de mélanges selon la revendication 1, pour préparer des additifs alimentaires.
